# FASCICULE DE BREVET EUROPEEN

(11) **EP 2 844 182 B1**
(45) Date de publication et mention de la délivrance du brevet: **05.09.2018**
(21) Numéro de dépôt: 13785283.6
(22) Date de dépôt: 29.04.2013
(51) Int. Cl.: A61C 1/08, A61B 5/00, A61B 5/107

(54) **Dispositif intra-buccal pour la preparation automatisée des dents en vue de réaliser des restaurations partielles ou périphériques**
Intraorale Vorrichtung zur automatisierten Präparation der Zähne für partielle oder periphere Restaurationen
Intraoral Device for Automated Preparation of The Teeth in Order to Perform Partial or Peripheral Restorations

(30) Priorité: 04.05.2012 FR 1254147
(43) Date de publication de la demande: 11.03.2015
(73) Titulaire: Koubi, Stephen, 13001 Marseille (FR); Gurel, Galip, 34367 Istanbul (TR)
(72) Inventeur: Koubi, Stephen, 13001 Marseille (FR); Gurel, Galip, 34367 Istanbul (TR)
(74) Mandataire: Roman, Alexis
(86) Numéro de dépôt international: PCT/FR2013/050948
(87) Numéro de publication internationale: WO 2013/164537

(56) Documents cités:
- WO-A1-94/03120
- FR-A1- 2 036 992
- US-A- 5 113 424
- US-A1- 2004 015 176

## Description

### Domaine technique de l'invention.

L'invention a pour objet un dispositif intra-buccal pour la préparation automatisée des dents en vue de réaliser des restaurations partielles ou périphériques.

L'invention concerne les techniques dentaires permettant de préparer une dent d'un patient en vue de réaliser une facette. Elle concerne plus particulièrement les appareils dentaires robotisés permettant de fraiser automatiquement en bouche, sans que le dentiste n'ait à manipuler l'outil de taille.

### État de la technique.

Une facette en céramique permet de recouvrir la face vestibulaire d'une dent tachée, décolorée, déformée ou légèrement détériorée par l'usure. Les facettes sont surtout placées sur les incisives centrales jusqu'aux premières prémolaires. Pour préparer une dent en vue de poser une facette, le dentiste doit enlever une fine couche d'émail sur la face vestibulaire de la dent. Une empreinte de la dent ainsi préparée est ensuite prise, puis transmise à un laboratoire pour confectionner les facettes.

L'inventeur a développé une technique permettant de minimiser la couche d'émail enlevée (« GUREL G. Predictable, precise, and repeatable tooth preparation for porcelain laminate veneers. Pract Proced Aesthet Dent. 2003; 15(1): 17-24 ». « GUREL G. Les facettes en céramiques : de la théorie à la pratique. Quitessence Publishing 2006 »). En premier lieu, le praticien prépare un projet esthétique correspondant à la forme finale de la dent et à son agencement sur l'arcade. Le projet esthétique est confectionné à partir d'une maquette en cire (ou « wax-up »). Il permet sa validation par le praticien et le patient, tant sur le plan esthétique que fonctionnel avant réalisation. Il va également servir de guide lors de la préparation. Le praticien doit ensuite préparer la dent en préservant un maximum d'émail (c'est-à-dire en évitant un enlèvement inutile de tissus) et en assurant au prothésiste une épaisseur constante pour construire la facette en céramique. En pratique, l'épaisseur de la facette céramique doit être d'au moins 0,5 mm sur la face vestibulaire et d'au moins 1,5 mm sur le bord incisif. Toute la difficulté consiste à préparer la dent a minima pour obtenir ces épaisseurs. Pour ce faire, une réplique du projet esthétique est appliquée en bouche. Le praticien fraise des gorges d'environ 0,5 mm de profondeur à travers le projet esthétique, sur la surface des dents. Il suffit alors, après avoir ôté le projet esthétique, d'égaliser la surface dentaire à préparer sans enlever plus de tissu dentaire que nécessaire. Une prise d'empreinte est de nouveau réalisée en utilisant la clé silicone ayant initialement servi à matérialiser le projet esthétique. C'est à partir de cette nouvelle empreinte que le prothésiste prépare la facette céramique sur mesure. Pour obtenir un résultat optimal, cette technique, notamment l'enlèvement du tissu dentaire et la prise d'empreinte qui suit, ne peut être réalisée que par des praticiens expérimentés.

Un objectif de l'invention est d'automatiser cette technique, de sorte qu'elle puisse être mise en oeuvre même par un praticien moins expérimenté.

Un autre objectif de l'invention est de proposer un appareil permettant de minimiser l'enlèvement de tissus dentaires.

Encore un objectif de l'invention est de proposer un appareil permettant d'améliorer la précision de la prise d'empreinte réalisée après la préparation de la dent.

Pour ce faire, un appareil dentaire robotisé a été développé par les inventeurs.

Les appareils dentaires robotisés sont bien connus de l'Homme du métier. On connait par exemple par le document brevet WO 94/03120 (BECKETT CORP LTD), un ensemble de dentisterie comprenant un outil de coupe associé à une gouttière. Le dentiste peut positionner l'outil de coupe en un emplacement désiré, par rapport à une dent d'un patient. L'outil reste toujours, de façon contrôlée, dans la position désirée, de telle sorte qu'un évidemment extrêmement précis peut être réalisé dans la dent, même en cas de mouvements de la tête du patient.

Ce type d'appareil ne permet pas des fraisages complexes et est inadapté pour la préparation d'une dent en vue de réaliser une facette selon la technique définie précédemment.

On connait également par le document brevet EP 0.345.975 (LORAN), un appareil comportant un outil de coupe déplacé le long d'une trajectoire prédéterminée. Le travail manuel du dentiste est préalablement réalisé sur un modèle, l'appareil étant fixé audit modèle dans une position fixe au moyen d'une gouttière. Les moteurs de déplacement de l'outil sont sollicités en réponse aux manipulations par le dentiste de crayons de commande de façon à déplacer ledit outil le long des trajectoires nécessaires pour obtenir la configuration désirée de la dent. Des signaux correspondant aux déplacements de l'outil sont délivrés à un ordinateur. Lorsque l'usinage est terminé sur le modèle, la restauration est préparée à partir de ce modèle et le patient est reconvoqué. L'appareil est alors fixé à la mâchoire du patient de manière a ce qu'il soit précisément dans la même position relative que celle qu'il occupait par rapport au modèle. Ensuite, l'ordinateur envoie des signaux aux moteurs de commande appropriés pour la réitération du déplacement de l'outil, respectant la séquence correcte préalablement enregistrée, de manière à reproduire les opérations d'usinage préalablement effectuées sur le modèle.

Cet appareil peut être adapté pour préparer une dent en vue de réaliser une facette selon la technique définie précédemment. Toutefois, si le travail manuel du dentiste initialement réalisé sur un modèle n'est pas de bonne qualité, l'appareil reproduira tous les défauts en bouche. L'objectif visé par l'invention ne peut donc être atteint par cet appareil.

Le document brevet US 2004/015176 (COSMAN) concerne un système de localiseur stéréostatique à impression dentaire permettant d'induire de manière non invasive des images de tranches tomographiques par ordinateur de l'anatomie d'un patient. Ce type d'appareil n'est pas adapté pour la préparation automatisée de dents en vue de réaliser une restauration partielle ou périphérique dentaire.

### Divulgation de l'invention.

La solution proposée par l'invention est un dispositif intra-buccal pour la préparation automatisée des dents en vue de réaliser des restaurations partielles ou périphériques, comprenant :
- une gouttière adaptée pour se positionner dans la bouche d'un patient, ladite gouttière comprenant des moyens pour la maintenir en position dans ladite bouche,
- au moins un outil de taille mobile associé à ladite gouttière,
- une unité de gestion électronique permettant de piloter ledit outil de taille.

Ce dispositif comprend les caractéristiques remarquables suivantes :
- ledit outil de taille est configuré pour tailler au moins la face vestibulaire de la dent à préparer, ledit outil étant monté sur un chariot mobile se déplaçant sur un rail fixé sur la gouttière, en vis-à-vis de la face vestibulaire de la dent à préparer, ledit rail ayant une courbure adaptée à la dentition du patient,
- ladite gouttière comprend au moins un outil de numérisation en 3D agencé de façon à numériser au moins la face vestibulaire de ladite dent à préparer, ledit outil de numérisation étant connecté à l'unité de gestion de manière à ce que les données numérisées puissent transiter vers ladite unité de gestion,
- ladite unité de gestion est configurée pour piloter le déplacement dudit outil de taille en fonction des données numérisées.

Grâce à cet appareil, il est maintenant possible d'automatiser la taille de la dent, de sorte que n'importe quel praticien, quel que soit son niveau d'expérience, puisse facilement mettre en oeuvre la technique développée par le Dr. GUREL. En effet, l'outil de taille est maintenant uniquement commandé en fonction des données scannées par l'outil de numérisation (c'est-à-dire en fonction de la morphologie de la face vestibulaire de la dent), et non plus en fonction de l'habileté technique et de la dextérité du praticien. Le fraisage peut donc être réalisé de manière très précise. Il en est de même de la prise d'empreinte qui est bien plus précise que celle réalisée à partir de la clé silicone ayant initialement servi à matérialiser le projet esthétique. L'optimisation de l'enlèvement de tissus dentaires combinée à la numérisation précise de la dent, permettent d'obtenir une facette ayant l'épaisseur minimale nécessaire à sa fabrication.

D'autres caractéristiques remarquables du dispositif intra-buccal objet de l'invention sont listées ci-dessous, chacune de ces caractéristiques pouvant être considérée seule ou en combinaison, indépendamment des caractéristiques remarquables définies ci-dessus :
- L'outil de taille est préférentiellement une fraise rotative montée sur le chariot mobile.
- L'outil de numérisation est préférentiellement monté sur un chariot mobile, ledit chariot se déplaçant sur un rail fixé sur la gouttière en vis-à-vis de la face vestibulaire de la dent à préparer, ledit rail ayant une courbure adaptée à la dentition du patient.

- L'outil de taille est préférentiellement monté de manière amovible sur un contre-angle intégrant une liaison rotule ou pivot offrant un débattement angulaire audit outil.
- Dans une variante de réalisation, l'outil de taille est un laser dentaire monté sur le chariot mobile.
- La gouttière peut être associée à un autre outil de taille mobile, ledit outil étant configuré pour tailler au moins le bord incisif de la dent à préparer ; ladite gouttière comprenant un autre outil de numérisation en 3D agencé de façon à numériser au moins le bord incisif de ladite dent à préparer, ledit autre outil de numérisation étant connecté à l'unité de gestion de manière à ce que les données numérisées puissent transiter vers ladite unité de gestion ; cette dernière étant configurée pour piloter le déplacement dudit autre outil de taille en fonction desdites données numérisées.
- Cet autre outil de taille est préférentiellement une fraise rotative montée sur un chariot mobile, ledit chariot se déplaçant sur un rail fixé sur la gouttière en vis-à-vis du bord incisif de la dent à préparer, ledit rail ayant une courbure adaptée à la dentition du patient.
- L'autre outil de numérisation est préférentiellement monté sur un chariot mobile, ledit chariot se déplaçant sur un rail fixé sur la gouttière en vis-à-vis du bord occlusal de la dent à préparer, ledit rail ayant une courbure adaptée à la dentition du patient.
- Dans une variante de réalisation, l'autre outil de taille est un laser dentaire monté sur un chariot mobile, ledit chariot se déplaçant sur un rail fixé sur la gouttière en vis-à-vis de la face vestibulaire de la dent à préparer, ledit rail ayant une courbure adaptée à la dentition du patient.

Un autre aspect de l'invention concerne une installation comprenant le dispositif selon l'une des caractéristiques précédentes et un centre d'usinage comprenant des outils pour usiner automatiquement une facette dentaire, l'unité de gestion dudit dispositif étant configurée pour piloter le déplacement desdits outils d'usinage en fonction des données numérisées par l'outil de numérisation 3D, lesdites données étant celles d'au moins la face vestibulaire de la dent à préparer et celle de ladite face vestibulaire une fois que ladite dent est préparée.

Encore un autre aspect de l'invention concerne une gouttière adaptée pour se positionner dans la bouche d'un patient, ladite gouttière comprenant des moyens pour la maintenir en position dans ladite bouche, au moins un outil de taille mobile étant associé à ladite gouttière, ledit outil étant configuré pour tailler au moins la face vestibulaire de la dent à préparer, ledit outil étant monté sur un chariot mobile se déplaçant sur un rail fixé sur la gouttière, en vis-à-vis de la face vestibulaire de la dent à préparer, ledit rail ayant une courbure adaptée à la dentition du patient, ladite gouttière comprenant en outre au moins un outil de numérisation en 3D agencé de façon à numériser au moins la face vestibulaire de ladite dent à préparer.

Encore un autre aspect de l'invention concerne un procédé pour piloter un outil de taille mobile associé à une gouttière adaptée pour se positionner dans la bouche d'un patient, ledit outil étant configuré pour tailler au moins la face vestibulaire d'une dent à préparer, ledit procédé consistant à :
- positionner ladite gouttière dans la bouche du patient et la maintenir en position,
- numériser en 3D au moins la face vestibulaire de ladite dent à préparer,
- piloter le déplacement dudit l'outil de taille en fonction des données numérisées.

Encore un autre aspect de l'invention concerne une technique de fabrication d'une facette dentaire consistant à :
- préparer un projet esthétique correspondant à la forme finale de la dent et à son agencement sur l'arcade.
- appliquer le projet esthétique sur la dent à préparer du patient,
- placer le dispositif objet de l'invention dans la bouche du patient,
- lancer la numérisation du projet esthétique en bouche,
- lancer le taillage d'au moins la face vestibulaire de la dent à préparer sur une profondeur prédéfinie et constante par rapport à la surface extérieure du projet esthétique en bouche,
- lancer la numérisation de la dent préparée,
- transmettre à un centre d'usinage les données numérisées de la dent préparée et celles du projet esthétique en bouche,
- fabriquer la facette en fonction des données numérisées reçues par le centre d'usinage.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 montre schématiquement un dispositif conforme à l'invention vue de dessus,
- la figure 2 montre schématiquement un dispositif conforme à l'invention vue de derrière,
- la figure 3 illustre l'agencement d'un dispositif conforme à l'invention par rapport à la dent à préparer, les outils de numérisation 3D étant disposés en vis-à-vis de ladite dent recouverte du projet esthétique,
- la figure 4 illustre l'agencement d'un dispositif conforme à l'invention par rapport à la dent à préparer, les outils de taille étant disposés en vis-à-vis de ladite dent recouverte du projet esthétique,
- la figure 5 montre le dispositif de la figure 2 avec les outils de taille en action,
- la figure 6 illustre l'agencement d'un dispositif conforme à l'invention par rapport à la dent préparée, les outils de numérisation 3D étant disposés en vis-à-vis de ladite dent,
- la figure 7 schématise la facette céramique destinée à être collée sur la dent préparée,
- la figure 8 illustre une installation conforme à l'invention,
- la figure 9 illustre l'agencement d'un dispositif conforme à l'invention par rapport à la dent à préparer, un seul outil permettant de tailler la face vestibulaire et le bord incisif de la dent à préparer.

### Modes préférés de réalisation de l'invention.

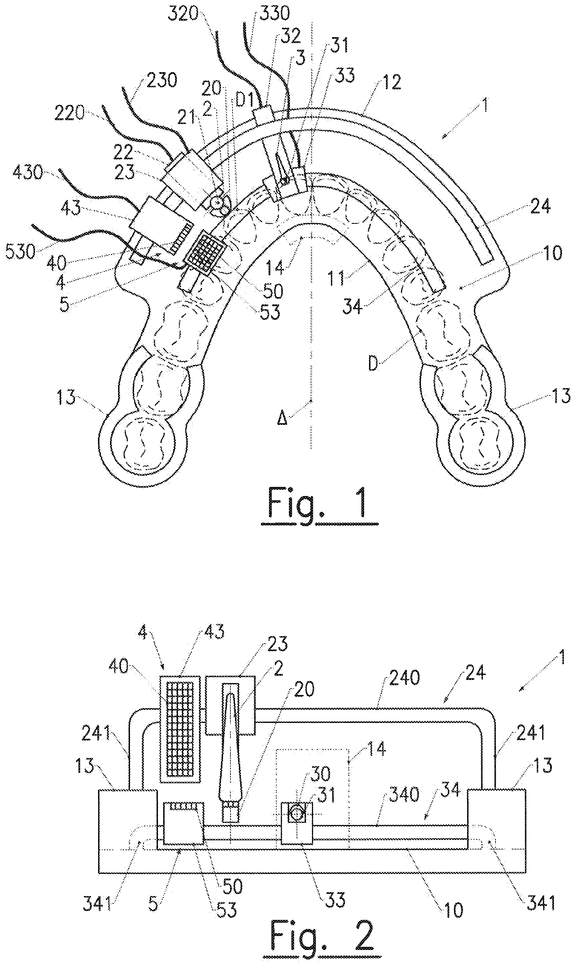

En se rapportant aux figures 1 et 2, le dispositif objet de l'invention comprend une gouttière 1 adaptée pour se positionner dans la bouche d'un patient. Cette gouttière 1 a une forme générale en « U » ou en « V » et présente un axe de symétrie par rapport au plan médian Δ du patient. Elle peut être obtenue par moulage plastique ou thermoformage. Elle comprend une zone de morsure 10 formée de manière à s'adapter à la courbure générale de la dentition D du patient. La zone de morsure 10 présente une zone d'occlusion disposée de manière à venir en contact de la dentition D lorsqu'elle est insérée dans la bouche du patient, un bord palatal 11 sur un côté proche de la gorge, et un bord labial 12 sur un autre côté proche des lèvres.

Pour maintenir la gouttière 1 en position dans la bouche du patient, il est prévu des crampons dentaires 13 solidaires de ladite gouttière. Ces crampons 13 sont disposés au niveau des secteurs molaires de la zone d'occlusion. Ils sont fixés sur les molaires de l'arcade dentaire à laquelle la dent à préparer D1 appartient. Pour éviter le basculement du bord labial 12 de la gouttière 1, il peut être avantageux de prévoir un élément en silicone disposé au milieu du bord palatal 11 et configuré de manière à prendre appui sur la face linguale des incisives centrales de l'arcade à laquelle la dent à préparer appartient. On pourrait également prévoir un autre crampon dentaire solidaire de la gouttière et configuré pour se fixer sur les incisives centrales. D'autres moyens pour maintenir la gouttière 1 en position dans la bouche du patient peuvent être utilisés. La gouttière peut par exemple comporter des zones de fixation destinées à être remplie de colle ou de matière plastique se solidifiant.

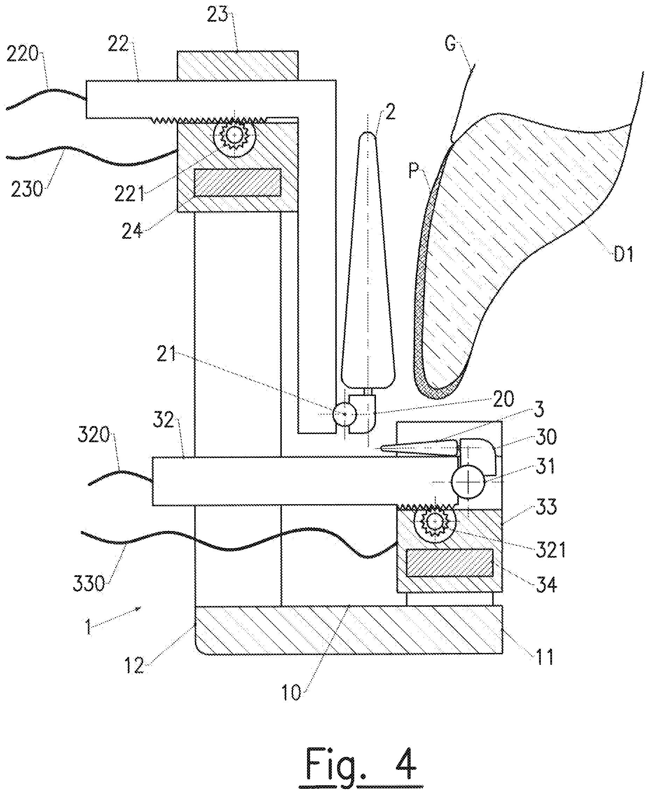

La gouttière 1 est associée à au moins un outil de taille mobile 2 configuré pour tailler au moins la face vestibulaire de la dent à préparer. En se rapportant aux figures 2 et 4, il s'agit préférentiellement d'un outil de taille rotatif du type fraise congé diamantée pouvant présenter une granulométrie normale (pour la réduction du volume dentaire) ou une faible granulométrie (polissage de la préparation) (d'autres modèles convenant à l'homme du métier pouvant être employés) montée de manière amovible sur un contre-angle 20, de la même façon que les outils de fraisage classiques de dentisterie. La longueur de la partie active de cette fraise congé est sensiblement égale, préférentiellement légèrement supérieure, à la longueur moyenne d'une dent. L'axe de rotation de l'outil 2 est initialement sensiblement vertical. L'outil de taille 2 est agencé sur la gouttière 1 de manière à ce qu'il puisse correctement atteindre et préparer la face vestibulaire de la dent à préparer et notamment ses limites cervicales.

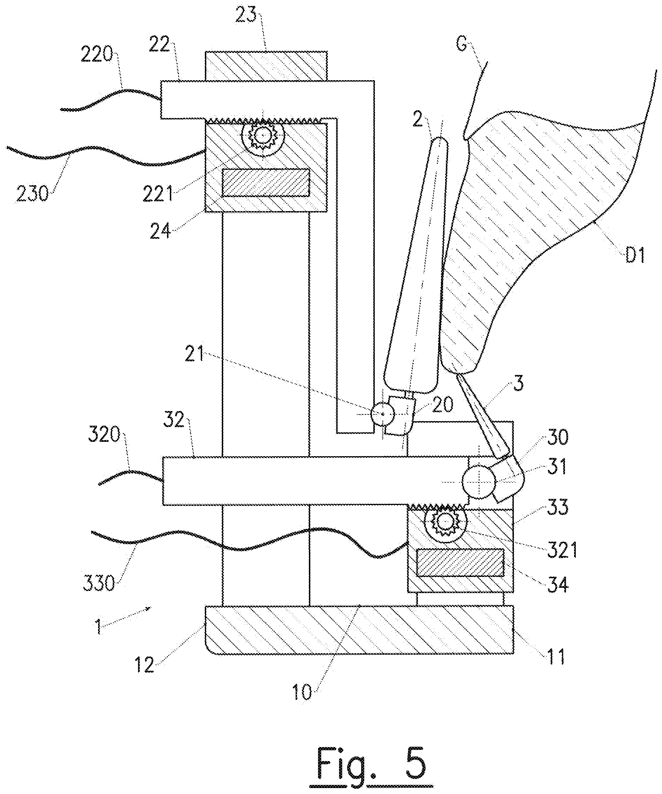

Le contre-angle 20 intègre une liaison rotule ou pivot 21. Cette liaison 21 offre un débattement angulaire à l'outil 2 (figure 5). Un premier moteur (non représenté) associé à la liaison 21 permet de régler l'inclinaison du contre-angle 20 et donc de l'angle de l'outil 2. Ce premier moteur consiste par exemple en un moteur électrique associé à un système de pignon/roue dentée permettant de modifier l'inclinaison du contre-angle 20. La liaison 21 est solidaire d'un manche 22 à l'intérieur duquel sont logés toutes les composants permettant de faire fonctionner l'outil de taille 2 et le premier moteur. Une pulvérisation d'eau et/ou d'air peut être prévue pour refroidir la zone de travail. De fait, le manche 22 comprend une connexion 220 électrique, hydraulique et/ou pneumatique prévu à cet effet.

Le manche 22 est monté sur un chariot mobile 23. Ce dernier est décrit plus en détail dans la suite de la description. En pratique le manche 22 est monté coulissant dans le chariot mobile 23, un second moteur 221 permettant de faire coulisser automatiquement ledit manche vers l'avant ou vers l'arrière, c'est-à-dire vers le bord palatal 11 ou vers le bord labial 12. Ce second moteur 221 consiste par exemple en un moteur électrique associé à un système de pignon/crémaillère permettant de déplacer le manche 22.

Le chariot mobile 23 se déplace sur un rail 24 fixé sur la gouttière 1. Le rail 24 est préférentiellement réalisé en inox. Il est disposé en vis-à-vis de la face vestibulaire de la dent D1 à préparer, sa courbure étant adaptée à la dentition D du patient. En se rapportant plus particulièrement aux figures 1 et 2, le rail 24 comprend une portion courbe 240 sensiblement verticale et dont les extrémités se terminent par deux jambages 241 fixés au niveau du bord labial 12 de la zone de morsure 10 de la gouttière 1. La portion courbe 240 est décalée de quelques millimètres de la zone de morsure 10 de manière à ce que le chariot mobile 23 soit situé sensiblement au niveau de la gencive G du patient (figure 4). Un troisième moteur (non représenté) permet de déplacer automatiquement le chariot mobile 23 sur le rail 24. Ce troisième moteur peut consister en un moteur électrique associé à un système de pignon/crémaillère permettant de déplacer le chariot mobile 23. Ce dernier comprend une connexion 230 électrique permettant de faire fonctionner le second et le troisième moteur.

L'outil de taille 2 comprenant donc au moins trois degrés de déplacement : un premier degré correspondant à l'inclinaison du contre-angle 20, un second degré correspondant au déplacement du manche 22 dans le chariot 23, et un troisième degré correspondant au déplacement dudit chariot sur le rail 24. C'est la raison pour laquelle les termes « outil articulé » ou « outil mobile » sont employés. D'autres déplacements peuvent bien évidemment être prévus. Ces différents déplacements sont contrôlés par les moteurs mentionnés précédemment pour que l'outil 2 taille au moins la face vestibulaire de la dent D1 à préparer, quelle que soit la morphologie de cette dernière et la quantité de tissus à enlever.

Dans une variante de réalisation non représentée, le taillage de la face vestibulaire de la dent D1 à préparer peut être effectué au moyen d'un laser dentaire apte à traiter des tissus durs tels que l'émail, par exemple un laser Erbium. Dans ce cas, l'outil de taille 2 se présente sous par exemple la forme d'un laser monté sur un chariot mobile 23 du type décrit précédemment. Ce laser présente des degrés de déplacement lui permettant de tailler la face vestibulaire de la dent D1 à préparer quelle que soit la morphologie de cette dernière et la quantité de tissus à enlever.

Selon la configuration de la facette à réaliser, il peut être nécessaire de tailler également le bord incisif de la dent D1 à préparer. Pour ce faire, il est envisagé d'associer la gouttière 1 à au moins un autre outil de taille 3 configuré pour tailler au moins le bord occlusal de la dent D1 à préparer. En se rapportant aux figures 1, 2 et 4, on préfère utiliser un outil de taille rotatif du type fraise congé diamantée montée de manière amovible sur un contre-angle 30. Compte tenu de la faible surface à tailler, la longueur de la partie active de cette fraise congé est moindre que celle de l'outil 2. Une fraise boule peut être employée de manière équivalente.

Le contre-angle 30 est pourvu d'une liaison rotule ou pivot 31. Cette liaison 31 autorise un débattement angulaire à l'outil 3 (figure 5). Un premier moteur (non représenté) associé à la liaison 31 permet de régler l'inclinaison du contre-angle 30 et donc de l'angle de l'outil 3. Ce premier moteur consiste par exemple en un moteur électrique associé à un système de pignon/roue dentée permettant de modifier l'inclinaison du contre-angle 30. La liaison 31 est solidaire d'un manche 32 à l'intérieur duquel sont logées toutes les connexions électriques, hydrauliques et/ou pneumatiques permettant de faire fonctionner l'outil de taille 3 et le premier moteur. Le manche 32 comprend une connexion 320 électrique, hydraulique et/ou prévue à cet effet. Une pulvérisation d'eau et/ou d'air peut notamment être prévue pour refroidir la zone de travail.

Le manche 32 est monté sur un chariot mobile 33. En pratique, le manche 32 est monté coulissant dans le chariot mobile 33, un second moteur 321 permettant de faire coulisser automatiquement ledit manche vers l'avant ou vers l'arrière, c'est-à-dire vers le bord palatal 11 ou vers le bord labial 12. Ce second moteur 221 consiste par exemple en un moteur électrique associé à un système de pignon/crémaillère permettant de déplacer le manche 32.

Le chariot mobile 33 se déplace sur un rail 34 fixé sur la gouttière 1. Le rail 34 est préférentiellement réalisé en inox. Il est disposé en vis-à-vis du bord incisif de la dent D1 à préparer, sa courbure étant adaptée à la dentition D du patient. En se rapportant plus particulièrement aux figures 1 et 2, le rail 34 comprend une portion courbe 340 sensiblement verticale et dont les extrémités se terminent par deux jambages 341 fixés sensiblement au milieu de la zone de morsure 10 de la gouttière 1. La portion courbe 340 est située sous le bord incisif de la dent D1 à préparer (figure 4). Elle est donc plus basse que la portion courbe 240 du rail 24, et plus proche du bord palatal 11. Un troisième moteur (non représenté) permet de déplacer automatiquement le chariot mobile 33 sur le rail 34. Ce troisième moteur peut consister en un moteur électrique associé à un système de pignon/crémaillère permettant de déplacer le chariot mobile 33. Ce dernier comprend une connexion 330 électrique permettant de faire fonctionner le second et le troisième moteur.

L'autre outil de taille 3 comprend donc également au moins trois degrés de déplacement : un premier degré correspondant à l'inclinaison du contre-angle 30, un second degré correspondant au déplacement du manche 32 dans le chariot 33, et un troisième degré correspondant au déplacement dudit chariot sur le rail 34. D'autres déplacements peuvent bien évidemment être envisagés. Ces différents déplacements sont contrôlés par les moteurs mentionnés précédemment pour que l'outil 3 taille au moins le bord incisif de la dent D1 à préparer, voire également une partie de sa face linguale, quelle que soit la morphologie de ladite dent et la quantité de tissus à enlever.

Dans une variante de réalisation non représentée, le taillage du bord incisif de la dent D1 à préparer peut être effectué au moyen d'un laser dentaire apte à traiter des tissus durs tels que l'émail, par exemple un laser Erbium. Dans ce cas, l'outil de taille 3 se présente sous par exemple la forme d'un laser monté sur un chariot mobile 33 du type décrit précédemment. Ce laser présente des degrés de déplacement lui permettant de tailler le bord incisif de la dent D1 à préparer quelle que soit la morphologie de cette dernière et la quantité de tissus à enlever. Il est à noter que le taillage de la face vestibulaire et du bord incisif de la dent D1 à préparer, peut être effectué au moyen d'un unique laser dentaire présentant des degrés de déplacement lui permettant un tel taillage.

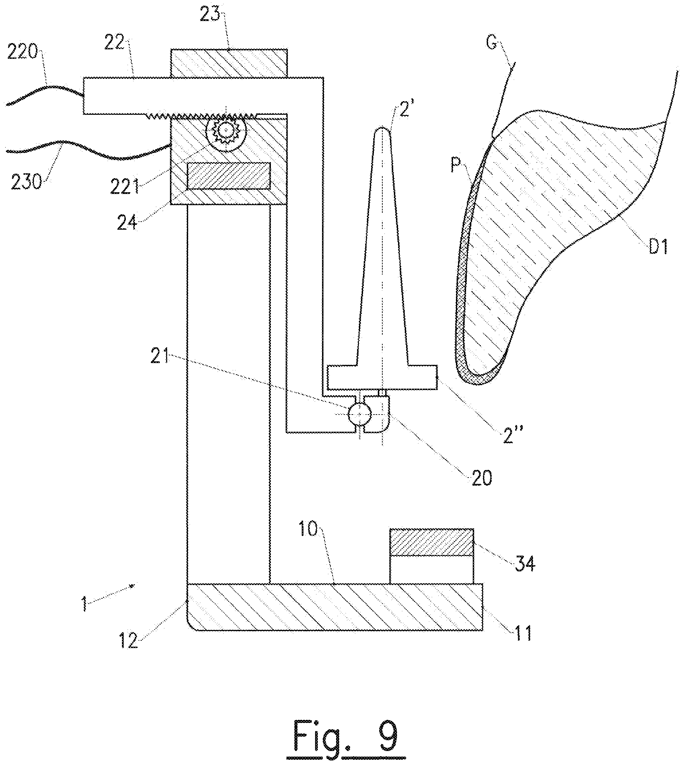

Selon encore une autre variante de réalisation représenté sur la figure 9, un unique outil de taille rotatif 2', 2" permet de préparer la face vestibulaire et le bord incisif de la dent D1. L'unité de gestion 6 ne gère donc maintenant que le déplacement d'un seul outil. Cet outil est similaire à l'outil 2 décrit en référence aux figures 4 et 5, et comprend le même nombre de degrés de déplacement. Il vient se fixer de manière amovible sur le contre-angle 20 décrit précédemment. Cet outil comprend une première portion 2' du type fraise congé diamantée. L'extrémité basse de cette première portion est pourvue d'une seconde portion cylindrique 2" diamantée. Cette seconde portion 2" est positionnée en vis-à-vis du bord incisif de la dent D1 à préparer. En déplaçant le manche 22, la seconde portion 2" est donc en mesure d'enlever des tissus au niveau du bord incisif de la dent D1.

En se rapportant à la figure 1, la présence des rails 24, 34 et des différents éléments qu'ils supportent, le bord labial 12 est avancé vers l'avant, dépassant généralement de la bouche du patient.

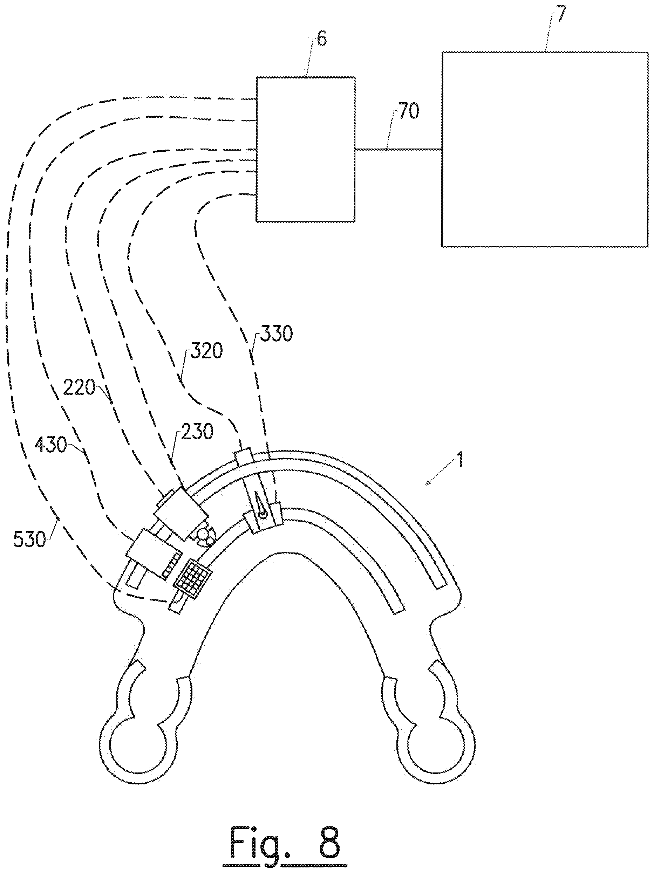

En se rapportant à la figure 8, les connexions 220, 230, 320 et 330 sont reliées à l'unité de gestion électronique 6, par une liaison filaire ou sans fil du type Wifi, Internet ou Bluetooth. En pratique, l'unité de gestion électronique 6 se présente sous la forme d'un ordinateur équipé d'un processeur, configuré pour exécuter un ou plusieurs programmes, sous-programmes, microprogrammes ou tous autres types de software équivalents, dont les instructions permettent de gérer l'inclinaison des contre-angles 20 et 30, le déplacement des manches 22 et 32, le déplacement des chariots 23 et 33, respectivement sur les rails 24 et 34, la mise en rotation des outils 2 et 3 ou leur arrêt. L'unité de gestion électronique 6 permet donc de piloter les outils de taille 2 et 3.

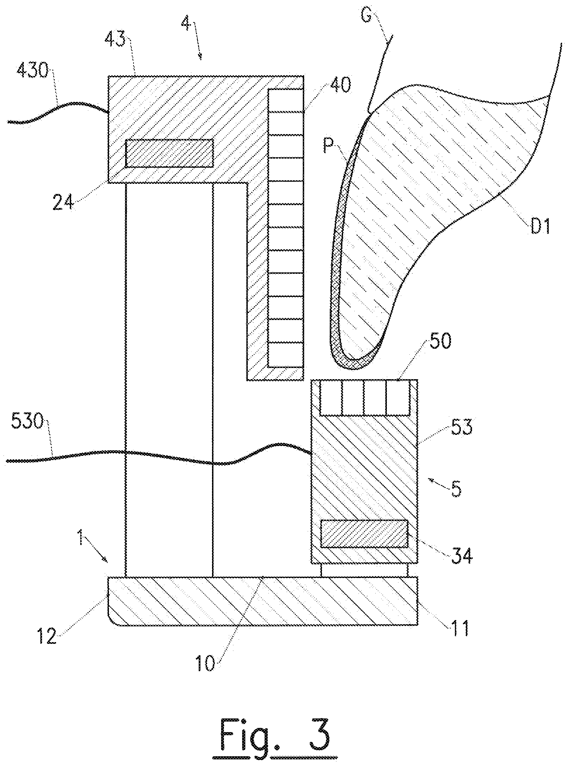

Conformément à l'invention, la gouttière 1 comprend au moins un outil 4 de numérisation en 3D. Cet outil consiste préférentiellement en une caméra capable de capturer des images en 3 dimensions. De tels outils de numérisation sont par exemple décrits dans les documents brevets US 2005/023781 (KNIGHTON) ou US 2004/0155975 (HART) ou commercialisées par la société 3M® sous la dénomination « caméra intra orale Lava® C.O.S. ». En se rapportant à la figure 3, l'outil de numérisation 4 est agencé sur la gouttière 1 de façon à numériser au moins la face vestibulaire de la dent D1 à préparer, c'est-à-dire recouverte du projet esthétique P. Pour cela, et comme représenté sur les figures 1 à 3, l'outil 4 comprend des capteurs 40 disposés verticalement, sensiblement sur toute la longueur moyenne et la largeur moyenne d'une dent.

L'outil de numérisation 4 est préférentiellement monté sur un chariot mobile 43. Dans un souci de compacité, le chariot mobile 43 se déplace préférentiellement sur le rail 24 décrit précédemment. Le rail 24 supporte donc non seulement le chariot 23 associé à l'outil de taille 2, mais également le chariot 43 associé à l'outil de numérisation 4. Il est toutefois envisageable de prévoir un autre rail similaire fixé sur la gouttière en vis-à-vis de la face vestibulaire de la dent D1 à préparer, ledit rail ayant une courbure adaptée à la dentition du patient. Un moteur (non représenté) permet de déplacer automatiquement le chariot mobile 43 sur le rail 24. Ce moteur peut consister en un moteur électrique associé à un système de pignon/crémaillère permettant de déplacer le chariot mobile 43. Ce dernier comprend une connexion électrique 430 permettant de faire fonctionner l'outil de numérisation 4.

La gouttière 1 peut comprendre un autre outil 5 de numérisation en 3D du type décrit précédemment. Toutefois, en se rapportant à la figure 3, cet autre outil de numérisation 5 est agencé sur la gouttière 1 de façon à numériser au moins le bord incisif de la dent D1 à préparer. Pour cela, et comme représenté sur les figures 1 à 3, l'autre outil 5 comprend des capteurs 50 disposés horizontalement, sensiblement sur toute la largeur moyenne et l'épaisseur moyenne d'une dent.

L'autre outil de numérisation 5 est préférentiellement monté sur un chariot mobile 53. Dans un souci de compacité, ce chariot mobile 53 se déplace préférentiellement sur le rail 34 décrit précédemment. Le rail 34 supporte donc non seulement le chariot 33 associé à l'outil de taille 3, mais également le chariot 53 associé à l'outil de numérisation 5. Il est toutefois envisageable de prévoir un autre rail similaire fixé sur la gouttière en vis-à-vis du bord incisif de la dent D1 à préparer, ledit rail ayant une courbure adaptée à la dentition du patient. Un moteur (non représenté) permet de déplacer automatiquement le chariot mobile 53 sur le rail 34. Le chariot mobile 53 comprend une connexion électrique 530 permettant de faire fonctionner l'outil de numérisation 5.

En se rapportant à la figure 8, les connexions 430 et 530 sont reliées à l'unité de gestion électronique 6, par une liaison filaire ou sans fil du type Wifi, Internet ou Bluetooth. Les données numérisées par les outils 4, 5 peuvent ainsi transiter vers l'unité de gestion 6. En pratique, l'unité de gestion électronique 6 intègre un ou plusieurs programmes, sous-programmes, microprogrammes ou tous autres types de software équivalents, dont les instructions permettent de gérer le déplacement des chariots 43 et 53 respectivement sur les rails 24 et 34, ainsi que l'activation/désactivation des outils 4, 5. Ces derniers sont donc pilotés par l'unité de gestion électronique 6.

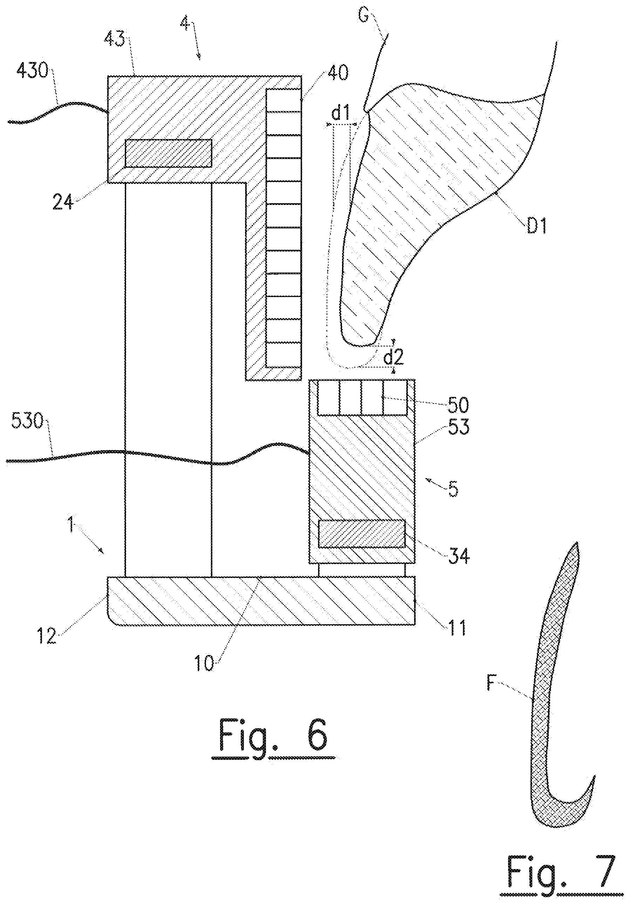

Selon une autre caractéristique remarquable de l'invention, l'unité de gestion 6 est configurée pour piloter au moins le déplacement du premier outil de taille 2 en fonction des données numérisées par le premier outil de numérisation 4. En pratique, l'unité de gestion 6 pilote également le déplacement du second outil de taille 3 en fonction des données numérisées par le second outil de numérisation 5. Et de manière plus générale, l'unité de gestion 6 pilote le déplacement des outils de taille 2, 3 en fonction des données numérisées par les outils de numérisation 4, 5 et donc en fonction de la morphologie de la dent à préparer. L'unité de gestion 6 peut ainsi piloter automatiquement le déplacement des outils de taille 2, 3, de sorte que ces derniers taillent de manière homogène la dent à préparer D1 (i.e. recouverte du projet esthétique P), sur une profondeur prédéfinie et constante : par exemple 0,2 mm à 0,5 mm (profondeur d1, figure 6) par rapport à la surface vestibulaire numérisée et 0 mm à 1,5 mm (profondeur d2, figure 6) par rapport au bord incisif numérisé. Le praticien est donc certain d'obtenir une micro-abrasion de la dent où seule la quantité de tissu dentaire nécessaire et suffisante à la réalisation de la facette est enlevée. La profondeur de préparation de la surface vestibulaire et/ou du bord incisif est choisie par le praticien en fonction de la situation clinique (par exemple, dans le cas d'une dent dyschromiée, l'épaisseur requise de la facette sera non pas de 0,5 mm comme généralement proposée, mais plutôt de 0,8 mm à 1 mm, afin que ladite facette puisse masquer la discoloration). En pratique, l'unité de gestion 6 propose au praticien de définir, via une interface s'affichant sur écran, la profondeur de pénétration des outils de préparation.

En résumé, le pilotage de l'outil de taille 2 (respectivement l'outil de taille 3) se fait en positionnant préalablement la gouttière 1 dans la bouche du patient et en la maintenant en position, puis en numérisant en 3D au moins la face vestibulaire (respectivement le bord incisif) de la dent D1 à préparer. Le pilotage du déplacement de l'outil est alors effectué en fonction des données numérisées.

Dans une variante de réalisation, on peut prévoir d'associer chaque outil de taille 2, 3 à un palpeur configuré pour mesurer l'enfoncement dudit outil dans la face vestibulaire et/ou dans le bord incisif de la dent D1 à préparer. Ce palpeur est connecté à l'unité de gestion 6 de manière à ce que les données relatives à la profondeur mesurée puissent transiter vers ladite unité de gestion. Cette dernière peut ainsi piloter automatiquement le déplacement de chaque outil de taille 2, 3, de sorte que ces derniers taillent de manière homogène la dent à préparer D1 recouverte du projet esthétique, sur une profondeur prédéfinie contrôlée par les palpeurs.

En se rapportant à la figure 8, le dispositif objet de l'invention peut être associé à un centre d'usinage 7 qui est situé dans le cabinet du praticien ou dans un autre lieu. Le centre d'usinage 7 consiste par exemple en une fraiseuse numérique CNC 4 axes pour prothèses dentaires, commercialisée sous la référence CHARLYDENTAL® 4X par la société CHARLYROBOT®. Ce type de centre d'usinage est bien connu de l'Homme du métier et comprend des outils aptes à usiner automatiquement une facette dentaire, notamment des facettes en céramique ou en résine. Le centre d'usinage 7 est relié à l'unité de gestion 6 par une liaison 70, filaire ou sans fil type Wifi, Internet ou Bluetooth de sorte que ladite unité puisse piloter le déplacement des outils d'usinage dudit centre en fonction des données numérisées par l'outil de numérisation 4 et/ou 5. Ces données numérisées sont celles d'au moins la face vestibulaire de la dent D1 à préparer (i.e. recouverte du projet esthétique P) et celle de ladite face vestibulaire une fois que ladite dent est préparée (i.e. après taillage). Ces données numérisées peuvent bien évidemment inclure celles relatives au bord incisif avant et après taillage. En effet, une fois que la dent D1 est taillée, cette dernière est de nouveau numérisée par les outils de numérisation 4, 5 (figure 6). En se rapportant à la figure 7, la forme et les dimensions de la facette F sont déduites en comparant l'image de la dent D1 à préparer (i.e. recouverte du projet esthétique P) avec l'image de la dite dent préparée (i.e. après taillage). L'unité de gestion 6 peut également sélectionner le type de plots de céramique à usiner (translucide ou opaque), en fonction de la situation clinique initiale et du projet esthétique.

En résumé, l'invention permet de mettre en oeuvre une nouvelle technique de fabrication d'une facette dentaire consistant à :
- placer le dispositif objet de l'invention dans la bouche du patient et lancer la numérisation de sa bouche ; une fois que cette numérisation initiale est réalisée, le dispositif peut être enlevé de la bouche du patient,
- préparer un projet esthétique correspondant à la forme finale de la dent et à son agencement sur l'arcade,
- appliquer le projet esthétique sur la dent à préparer du patient,
- placer le dispositif objet de l'invention dans la bouche du patient,
- lancer la numérisation du projet esthétique en bouche : figure 1, le ou les outils de numérisation 4, 5, sont déplacés sur leur rail respectif 24, 34, de manière à venir en vis-à-vis de la dent D1 à préparer,
- lancer le taillage d'au moins la face vestibulaire et éventuellement le bord incisif de la dent à préparer, sur une profondeur prédéfinie et constante par rapport à la surface extérieure du projet esthétique en bouche : figures 4 et 5, le ou les outils de numérisation 4, 5, sont écartés de la dent D1 à préparer pour laisser place aux outils de taille 2,3; ces derniers sont déplacés sur leur rail respectif 24, 34, de manière à venir en vis-à-vis de ladite dent,
- lancer la numérisation de la dent préparée : figure 6, les outils de taille 2,3 sont écartés de la dent D1 préparée pour laisser place aux outils de numérisation 4,5,
- transmettre à un centre d'usinage les données numérisées de la dent préparée et celles du projet esthétique en bouche,
fabriquer la facette en fonction des données numérisées reçues par le centre d'usinage.

Préalablement à l'étape de taillage, un périmètre de restauration peut être délimité par le praticien. Pour ce faire, l'unité de gestion 6 est reliée à un écran sur lequel s'affiche l'image de la dent à restaurer. A l'aide d'une souris ou d'un autre organe de pointage, le praticien délimite avec précision le périmètre de restauration sur l'image initiale (sans projet) de la dent à restaurer. L'unité de gestion 6 prend en compte ce périmètre de manière à ce que les outils de taille 2,3 n'enlèvent le tissus dentaire qu'à l'intérieur dudit périmètre.

## Revendications

1. Dispositif intra-buccal pour la préparation automatisée de dents en vue de réaliser une restauration partielle ou périphérique dentaire, comprenant :
- une gouttière (1) adaptée pour se positionner dans la bouche d'un patient, ladite gouttière comprenant des moyens (13) pour la maintenir en position dans ladite bouche,
- au moins un outil de taille (2) mobile associé à la gouttière (1),
- une unité de gestion électronique (6) permettant de piloter l'outil de taille (2),
**se caractérisant par le fait que** :
- l'outil de taille (2) est configuré pour tailler au moins la face vestibulaire de la dent (D1) à préparer, ledit outil étant monté sur un chariot mobile (23) se déplaçant sur un rail (24) fixé sur la gouttière (1), en vis-à-vis de la face vestibulaire de la dent (D1) à préparer, ledit rail ayant une courbure adaptée à la dentition (D) du patient,
- la gouttière (1) comprend au moins un outil (4) de numérisation en 3D agencé de façon à numériser au moins la face vestibulaire de ladite dent à préparer, ledit outil de numérisation étant connecté à l'unité de gestion (6) de manière à ce que les données numérisées transitent vers ladite unité de gestion,
- l'unité de gestion (6) pilotant le déplacement dudit outil de taille (2) en fonction des données numérisées.

2. Dispositif selon la revendication 1, dans lequel l'outil de taille (2) est une fraise rotative.

3. Dispositif selon l'une des revendications précédentes, dans lequel l'outil de numérisation (4) est monté sur un chariot mobile (43), ledit chariot se déplaçant sur un rail (24) fixé sur la gouttière (1) en vis-à-vis de la face vestibulaire de la dent (D1) à préparer, ledit rail ayant une courbure adaptée à la dentition (D) du patient.

4. Dispositif selon l'une des revendications précédentes, dans lequel l'outil de taille (2) est monté de manière amovible sur un contre-angle (20) intégrant une liaison rotule ou pivot (21) offrant un débattement angulaire audit outil.

5. Dispositif selon la revendication 1, dans lequel l'outil de taille (2) est un laser dentaire.

6. Dispositif selon l'une des revendications précédentes, dans lequel :
- la gouttière (1) est associée à un autre outil de taille mobile (3), ledit outil étant configuré pour tailler au moins le bord incisif de la dent (D1) à préparer,
- ladite gouttière comprend un autre outil (5) de numérisation en 3D agencé de façon à numériser au moins le bord incisif de ladite dent à préparer, ledit autre outil de numérisation étant connecté à l'unité de gestion (6) de manière à ce que les données numérisées puissent transiter vers ladite unité de gestion,
- ladite unité de gestion (6) est configurée pour piloter le déplacement dudit autre outil de taille en fonction desdites données numérisées.

7. Dispositif selon la revendication 6, dans lequel l'autre outil de taille mobile (3) est une fraise rotative montée sur un chariot mobile (33), ledit chariot se déplaçant sur un rail (34) fixé sur la gouttière (1) en vis-à-vis du bord incisif de la dent (D1) à préparer, ledit rail ayant une courbure adaptée à la dentition (D) du patient.

8. Dispositif selon la revendication 6, dans lequel l'autre outil de taille mobile (3) est un laser dentaire monté sur un chariot mobile (33), ledit chariot se déplaçant sur un rail (34) fixé sur la gouttière (1) en vis-à-vis du bord incisif de la dent (D1) à préparer, ledit rail ayant une courbure adaptée à la dentition (D) du patient.

9. Dispositif selon l'une des revendications 6 à 8, dans lequel l'autre outil de numérisation (5) est monté sur un chariot mobile (53), ledit chariot se déplaçant sur un rail (34) fixé sur la gouttière (1) en vis-à-vis du bord incisif de la dent (D1) à préparer, ledit rail ayant une courbure adaptée à la dentition (D) du patient.

10. Installation **se caractérisant par le fait qu'**elle comprend le dispositif selon l'une des revendications 1 à 9 et un centre d'usinage (7) comprenant des outils pour usiner automatiquement une facette dentaire, l'unité de gestion (6) dudit dispositif pilotant le déplacement desdits outils d'usinage en fonction des données numérisées par l'outil (4) de numérisation 3D, lesdites données étant celles d'au moins la face vestibulaire de la dent (D1) à préparer et celle de ladite face vestibulaire une fois que ladite dent est préparée.

## Patentansprüche

1. Intraorale Vorrichtung zum automatisierten Vorbereiten von Zähnen für das Durchführen einer partiellen oder peripheren Zahnrestauration, umfassend:
- eine zum Anordnen im Mund eines Patienten ausgebildete Schiene (1), wobei die Schiene Mittel (13) umfasst, diese im Mund in Position zu halten,
- wenigstens ein mit der Schiene (1) verbundenes bewegliches Schneidwerkzeug (2),
- eine das Steuern des Schneidwerkzeugs (2) ermöglichende elektronische Steuereinheit (6),
**dadurch gekennzeichnet, dass**:
- das Schneidwerkzeug (2) zum Schneiden wenigstens der vestibulären Fläche des vorzubereitenden Zahns (D1) ausgebildet ist, wobei das Werkzeug auf einem sich auf einer auf der Schiene (1) befestigten Schiene (24) gegenüber der vestibulären Seite des vorzubereitenden Zahns (D1) verschiebenden beweglichen Schlitten (23) montiert ist, wobei die Schiene (24) eine an das Gebiss (D) des Patienten angepasste Krümmung aufweist,
- die Schiene (1) wenigstens ein zum Digitalisieren wenigstens der vestibulären Fläche des vorzubereitenden Zahns ausgebildetes Werkzeug (4) zum 3D-Digitalisieren umfasst, wobei das Digitalisierungswerkzeug mit der Steuereinheit (6) verbunden ist, so dass die digitalisierten Daten zur Steuereinheit übertragen werden,
- wobei die Steuereinheit (6) das Verschieben des Schneidwerkzeugs (2) entsprechend den digitalisierten Daten steuert.

2. Vorrichtung nach Anspruch 1, wobei das Schneidwerkzeug (2) eine Rotationsfräse ist.

3. Vorrichtung nach einem der vorhergehenden Ansprüche, wobei das Digitalisierungswerkzeug (4) auf einem beweglichen Schlitten (43) montiert ist, wobei sich der Schlitten auf einer auf der Schiene (1) befestigten Schiene (24) gegenüber der vestibulären Seite des vorzubereitenden Zahns (D1) verschiebt, wobei die Schiene eine an das Gebiss (D) des Patienten angepasste Krümmung aufweist.

4. Vorrichtung nach einem der vorhergehenden Ansprüche, wobei das Schneidwerkzeug (2) abnehmbar auf einem Gegenwinkel (20) umfassend eine Dreh- oder Schwenkverbindung (21) zum Ermöglichen einer Winkelbewegung des Werkzeugs montiert ist.

5. Vorrichtung nach Anspruch 1, wobei das Schneidwerkzeug (2) ein Zahnlaser ist.

6. Vorrichtung nach einem der vorhergehenden Ansprüche, wobei:
- die Schiene (1) mit einem weiteren beweglichen Schneidwerkzeug (3) verbunden ist, wobei das Werkzeug zum Schneiden wenigstens der Bisskante des vorzubereitenden Zahns (D1) ausgebildet ist,
- die Schiene ein weiteres zum Digitalisieren wenigstens der Bisskante des vorzubereitenden Zahns ausgebildetes Werkzeug (5) zum 3D-Digitalisieren umfasst, wobei das weitere Digitalisierungswerkzeug mit der Steuereinheit (6) verbunden ist, so dass die digitalisierten Daten zur Steuereinheit übertragen werden können,
- wobei die Steuereinheit (6) zum Steuern des Verschiebens des weiteren Schneidwerkzeugs entsprechend den digitalisierten Daten ausgebildet ist.

7. Vorrichtung nach Anspruch 6, wobei das weitere bewegliche Schneidwerkzeug (3) eine auf einem beweglichen Schlitten (33) montierte Rotationsfräse ist, wobei sich der Schlitten auf einer auf der Schiene (1) gegenüber der Bisskante des vorzubereitenden Zahns (D1) befestigten Schiene (34) verschiebt, wobei die Schiene eine an das Gebiss (D) des Patienten angepasste Krümmung aufweist.

8. Vorrichtung nach Anspruch 6, wobei das weitere bewegliche Schneidwerkzeug (3) ein auf einem beweglichen Schlitten (33) montierter Zahnlaser ist, wobei sich der Schlitten auf einer auf der Schiene (1) gegenüber der Bisskante des vorzubereitenden Zahns (D1) befestigten Schiene (34) verschiebt, wobei die Schiene eine an das Gebiss (D) des Patienten angepasste Krümmung aufweist.

9. Vorrichtung nach einem der Ansprüche 6 bis 8, wobei das weitere Digitalisierungswerkzeug (5) auf einem beweglichen Schlitten (53) montiert ist, wobei sich der Schlitten auf einer auf der Schiene (1) befestigten Schiene (34) gegenüber der Bisskante des vorzubereitenden Zahns (D1) verschiebt, wobei die Schiene eine an das Gebiss (D) des Patienten angepasste Krümmung aufweist.

10. Anlage, **dadurch gekennzeichnet, dass** sie die Vorrichtung nach einem der Ansprüche 1 bis 9 und ein Bearbeitungszentrum (7) umfassend Werkzeuge zur automatischen Bearbeitung einer Zahnfacette umfasst, wobei die Steuereinheit (6) der Vorrichtung das Verschieben der Bearbeitungswerkzeuge entsprechend den vom Werkzeug (4) zur 3D-Digitalisierung digitalisierten Daten steuert, wobei die Daten von wenigstens der vestibulären Fläche des vorzubereitenden Zahns (D1) und der vestibulären Fläche nach dem Vorbereiten des Zahns stammen.

## Claims

1. Intrabuccal device for the automated preparation of teeth with a view to performing a partial or peripheral dental restoration, comprising:
- a splint (1) suitable for being positioned in the mouth of a patient, said splint comprising means (13) for holding it in position in said mouth;
- at least one movable cutting tool (2) associated with the splint (1);
- an electronic management unit (6) allowing the cutting tool (2) to be controlled,
being **characterized in that**:
- the cutting tool (2) is configured to cut at least the vestibular face of the tooth (D1) to be prepared, said tool being mounted on a movable carriage (23) moving on a rail (24) that is attached to the splint (1), facing the vestibular face of the tooth (D1) to be prepared, the curvature of said rail being adapted to the dentition (D) of the patient;
- the splint (1) comprises at least one 3D digitization tool (4) that is arranged so as to digitize at least the vestibular face of said tooth to be prepared, said digitization tool being connected to the management unit (6) so that the digitized data are conveyed to said management unit;
- the management unit (6) controlling the movement of said cutting tool (2) according to the digitized data.

2. Device according to Claim 1, in which the cutting tool (2) is a rotary burr.

3. Device according to one of the preceding claims, in which the digitization tool (4) is mounted on a movable carriage (43), said carriage moving on a rail (24) that is attached to the splint (1) facing the vestibular face of the tooth (D1) to be prepared, the curvature of said rail being adapted to the dentition (D) of the patient.

4. Device according to one of the preceding claims, in which the cutting tool (2) is removably mounted on a contra-angle (20) incorporating a ball or pivot joint (21) providing said tool with an angular excursion.

5. Device according to Claim 1, in which the cutting tool (2) is a dental laser.

6. Device according to one of the preceding claims, in which:
- the splint (1) is associated with another movable cutting tool (3), said tool being configured to cut at least the incisal edge of the tooth (D1) to be prepared;
- said splint comprises another 3D digitization tool (5) that is arranged so as to digitize at least the incisal edge of said tooth to be prepared, said other digitization tool being connected to the management unit (6) so that the digitized data can be conveyed to said management unit;
- said management unit (6) is configured to control the movement of said other cutting tool according to said digitized data.

7. Device according to Claim 6, in which the other movable cutting tool (3) is a rotary burr mounted on a movable carriage (33), said carriage moving on a rail (34) that is attached to the splint (1) facing the incisal edge of the tooth (D1) to be prepared, the curvature of said rail being adapted to the dentition (D) of the patient.

8. Device according to Claim 6, in which the other movable cutting tool (3) is a dental laser mounted on a movable carriage (33), said carriage moving on a rail (34) that is attached to the splint (1) facing the incisal edge of the tooth (D1) to be prepared, the curvature of said rail being adapted to the dentition (D) of the patient.

9. Device according to one of Claims 6 to 8, in which the other digitization tool (5) is mounted on a movable carriage (53), said carriage moving on a rail (34) that is attached to the splint (1) facing the incisal edge of the tooth (D1) to be prepared, the curvature of said rail being adapted to the dentition (D) of the patient.

10. Setup **characterized by** the fact that it comprises the device according to one of Claims 1 to 9 and a machining centre (7) comprising tools for automatically machining a dental facet, the management unit (6) for managing said device controlling the movement of said machining tools according to the data digitized by the 3D digitization tool (4), said data being those of at least the vestibular face of the tooth (D1) to be prepared and that of said vestibular face once said tooth has been prepared.
